# FASCICULE DE BREVET EUROPEEN

(11) **EP 1 530 584 B1**
(45) Date de publication et mention de la délivrance du brevet: **26.08.2009**
(21) Numéro de dépôt: 02774847.4
(22) Date de dépôt: 26.07.2002
(51) Int. Cl.: C07K 14/16, C12N 9/12, C12N 9/16, A61K 38/16, A61K 38/45, A61K 38/46, C12N 15/48, C12N 15/54, C12N 15/55, G01N 33/68, A61P 31/12, A61P 33/00

(54) **PEPTIDES SYNTHETIQUES OU NATURELS LIANT LA PROTEINE PHOSPHATASE 2A, METHODE D'IDENTIFICATION ET UTILISATIONS**
AN PROTEINPHOSPHATASE 2A BINDENDE SYNTHETISCHE ODER NATÜRLICHE PEPTIDE, IDENTIFIKATIONSVERFAHREN UND ANWENDUNGEN
SYNTHETIC OR NATURAL PEPTIDES BINDING PROTEIN PHOSPHATASE 2A, IDENTIFICATION METHOD AND USES

(30) Priorité: 27.07.2001 FR 0110139
(43) Date de publication de la demande: 18.05.2005
(73) Titulaire: INSTITUT PASTEUR, 75724 Paris Cedex 15 (FR); INSTITUT NATIONAL DE LA RECHERCHE AGRONOMIQUE, 75341 Paris Cédex 07 (FR); CONSEJO SUPERIOR DE INVESTIGACIONES CIENTIFICAS, E-28006 Madrid (ES); CENTRE NATIONAL DE LA RECHERCHE SCIENTIFIQUE (CNRS), 75794 Paris Cedex 16 (FR)
(72) Inventeur: GARCIA, Alphonse, 92120 Montrouge (FR); CAYLA, Xavier, 37210 Vernon sur Brenne (FR); REBOLLO, Angelita, 28039 Madrid (ES); LANGSLEY, Gordon, 75015 Paris (FR)
(74) Mandataire: Desaix, Anne
(86) Numéro de dépôt international: PCT/FR2002/002705
(87) Numéro de publication internationale: WO 2003/011898

(56) Documents cités:
- WO-A-01/04629
- WO-A-98/01563
- WO-A1-01/32867
- WO-A2-01/90159
- FAULKNER N E ET AL: "Protein phosphatase 2A activates the HIV-2 promoter through enhancer elements that include the pets site." THE JOURNAL OF BIOLOGICAL CHEMISTRY. UNITED STATES 13 JUL 2001, vol. 276, no. 28, 13 juillet 2001 (2001-07-13), pages 25804-25812, XP002201695 ISSN: 0021-9258
- DATABASE EMBL [Online] VPR Protein (fragment) 1 mai 1997 (1997-05-01), XP002201698 Database accession no. P89821
- DATABASE EMBL [Online] Ca2+/Calmodulin-dependent Protein Kinase V (frag 1 mai 2000 (2000-05-01), XP002201699 Database accession no. Q9QV79
- MORENO C S ET AL: "WD40 repeat proteins striatin and S/G(2) nuclear autoantigen are members of a novel family of calmodulin-binding proteins that associate with protein phosphatase 2A." THE JOURNAL OF BIOLOGICAL CHEMISTRY. UNITED STATES 25 FEB 2000, vol. 275, no. 8, 25 février 2000 (2000-02-25), pages 5257-5263, XP002201696 ISSN: 0021-9258
- MORENO C S ET AL: "A mammalian homolog of yeast MOB1 is both a member and a putative substrate of striatin family-protein phosphatase 2A complexes." THE JOURNAL OF BIOLOGICAL CHEMISTRY. UNITED STATES 29 JUN 2001, vol. 276, no. 26, 29 juin 2001 (2001-06-29), pages 24253-24260, XP002201697 ISSN: 0021-9258

## Description

L'invention a trait à de nouveaux peptides, synthétiques ou naturels utiles en particulier dans le traitement des infections virales ou parasitaires ou dans le traitement de tumeurs, lesdits peptides étant d'une taille inférieure à 30 acides aminés, de préférence inférieure à 20 acides aminés, notamment de 15 à 20 acides aminés, et caractérisés en ce qu'ils lient, *in vitro*, de manière spécifique, une holoenzyme protéine phosphatase de type 2A ou l'une de ses sous-unités. L'invention a aussi trait à une méthode d'identification de tels peptides, et à leurs utilisations.

Etant donné le rôle des peptides de l'invention dans la modulation de l'activité de la protéine phosphatase 2A cellulaire, il est important de rappeler en introduction les connaissances actuelles sur les protéine phosphatases 2A, leur rôle physiologique et leurs interactions avec certaines protéines cellulaires, virales ou parasitaires.

La physiologie de la cellule est contrôlée en partie par la modulation de l'état de phosphorylation des protéines. L'état de phosphorylation des protéines cellulaires dépend de l'action antagoniste des protéine kinases qui les phosphorylent et des protéine phosphatases qui les déphosphorylent.

Les protéines phosphatases sont divisées en deux groupes principaux : les tyrosine phosphatases et les sérine/thréonine phosphatases. Les sérine/thréonine phosphatases sont classées en deux catégories selon la spécificité de leur substrat et leur sensibilité à certains inhibiteurs, les phosphatases de type 1 (PP1) et les phosphatases de type 2 (PP2). Les phosphatases de type 2 se divisent encore en différentes classes, incluant la phosphatase 2A (PP2A), la phosphatase 2B ou calcineurine dont l'activité est régulée par le calcium, et la phosphatase 2C (PP2C) dont l'activité est régulée par le magnésium.

On sait maintenant que les phosphatases de type 2A sont très conservées au cours de l'évolution et sont potentiellement impliquées dans la régulation de nombreux processus biologiques. Les enzymes PP2A ont clairement été impliquées dans la régulation de la transcription, le contrôle du cycle cellulaire ou de la transformation virale. En outre, les PP2A sont la cible de différentes protéines virales ou parasitaires, suggérant un rôle des PP2A dans les interactions hôtes-pathogènes.

Les PP2A sont des complexes oligomèriques (holoenzymes) comprenant chacun, une sous-unité catalytique (C) et une ou deux sous-unités régulatrices, (A) et (B). La structure de la sous-unité (A) consiste en 15 répétions imparfaites d'une séquence d'acides aminés conservée de 38 à 40 acides aminés, dont certaines interagissent avec les sous-unités (B) et (C). Les sous-unités (A) et (C), conservées au cours de l'évolution, constituent la structure de base de l'enzyme et sont exprimées constitutivement. Au contraire, les sous-unités (B) constituent une famille de protéines régulatrices non reliées par une structure commune et différentiellement exprimées (Cohen P. The structure and regulation of protein phosphatases. Annu Rev Biochem 1989 ; 58 : 453-508). Ainsi, Les protéine phosphatase 2A existent *in vivo* sous deux classes de forme différente : une forme dimérique (AC) et une forme trimérique (ABC). Les sous-unités (B) régulent l'activité phosphatasique et la spécificité vis-à-vis du substrat. L'existence de formes multiples de PP2A est corrélée à des fonctions distinctes et variées des PP2A *in vivo.*

Récemment, différentes protéines synthétisées par des pathogènes, et en particulier des protéines virales et parasitaires ont été impliquées dans la modulation de certaines activités spécifiques des protéine phosphatase 2A.

Différentes stratégies impliquant PP2A ont été adoptées par les virus pour faciliter leur réplication et leur survie dans la cellule hôte. Par exemple, le *parainfluenza* virus incorpore dans sa particule virale la protéine PKC ζ, protéine d'origine cellulaire sous le contrôle de PP2A. Ceci lui permet de perturber la phosphorylation des protéines de son hôte et de faciliter sa propre réplication (De BP, Gupta S., Barnejee AK. Cellular protein kinase C ξ regulates human parainfluenza virus type 3 replication. Proc. Natl. Acad Sci USA 1995 ; 92 :5204-8).

Plusieurs virus à ADN ayant un pouvoir transformant, tels que les *papovae* ou les adénovirus, de même que certains rétrovirus, tels que le virus de l'immunodéficience humaine type 1 (VIH-1) codent pour des protéines qui interagissent directement avec certaines PP2A de l'hôte. Tous ces virus comprennent des protéines qui bien que structurellement différentes, interagissent avec certaines holoenzymes et en modifient l'activité phosphatasique.

Il a été montré en particulier que la protéine E4orf4 des adénovirus se lie à une PP2A hétérotrimèrique, et plus précisément à une sous-unité régulatrice (B), ce qui entraîne une diminution de la transcription de JunB dans la cellule infectée. Cet effet pourrait jouer un rôle important durant l'infection virale en régulant la réponse apoptotique des cellules infectées. De manière intéressante, il a aussi été montré que l'interaction de E4orf4 avec PP2A induit l'apoptose des cellules transformées d'une manière p53-indépendante (Shtrichman R.et al. Adenovirus type 5 E4 open reading frame 4 protein induces apotosis in transformed cells. J. Virol. 1998 ; 72 : 2975-82).

Les virus générant des tumeurs de la famille des *Papovae*, incluant SV40 et le virus du polyome, induisent la transformation cellulaire. Il a été montré que PP2A interagit avec l'antigène « petit T » de SV40 ou du polyome ainsi qu'avec la protéine transformante « moyen T » du polyome. Ces interactions de protéines virales avec PP2A ont été clairement impliquées dans la transformation virale. Enfin, la régulation transcriptionnelle, un processus normalement réalisé dans la cellule par les différents facteurs se fixant spécifiquement sur des séquences régulatrices promotrices, représente probablement le mécanisme le plus important impliqué dans le contrôle de l'expression virale par PP2A. Ainsi, il a été démontré que PP2A est un régulateur négatif de nombreux facteurs de transcription impliqués notamment dans les processus de croissance et de prolifération cellulaire, incluant AP1/SRE, NF-κB, Sp1 et CREB (Waszinski, B.E., Wheat W. H., Jaspers S., Peruski L.F., JR Lickteig R.L., Johnson G.L., and Klemm D.J. Nuclear protein phosphatase 2A dephosphorylates protein kinase A-phosphorylated CREB and regulates CREB Transcriptional stimulation. Mol Cell Biol. 1993 13, 2822-34). La régulation virale de ces facteurs de transcription permettrait de moduler la transcription virale.

La protéine virale de VIH-1, *Vpr*, interagit *in vitro* avec PP2A et stimule l'activité catalytique de PP2A (Tung L, et al, Direct activation of protein phosphatase 2A0 by HIV-1 encoded protein complex Ncp7 :vpr. FEBS Lett 1997 ; 401 : 197-201). *Vpr* peut induire l'arrêt en G2 des cellules infectées en inhibant l'activation du complexe p34cdc2-cycline B. D'autre part, *Vpr* interagit avec le facteur de transcription Sp1 et est un trans-activateur faible de la transcription de VIH-1 Sp1 dépendante. Ainsi, la protéine *Vpr* de VIH-1, qui est incorporée dans le virion, serait impliquée *in vivo* dans l'initiation de la transcription virale, une étape évidemment essentielle pour réguler l'expression du facteur de transcription Tat (un régulateur majeur de la transcription codé par le virus VIH-1).

Au contraire du rôle bien établi des protéine kinases dans les infections parasitaires, c'est seulement au cours de ces trois dernières années que les sérine/thréonine phosphatases ont commencé à être reconnues comme étant des régulateurs potentiels importants dans le domaine de la parasitologie.

Initialement, deux sérine-thréonine phosphatases, Ppβ et PfPP ont été identifiées dans *Plasmodium falciparum*. La présence d'activité phosphatasique de type 1 et de type 2A dans le parasite a été démontrée par des études enzymologiques. Dernièrement, des enzymes parasitaires PP2A et PP2B ont été purifiées.

Les sérine/thréonine phosphatases ont été récemment étudiées chez *Theileria parva,* un autre protozoaire proche de *P. falciparum* qui parasite les bovins. Les cellules hôtes, monocytes et leucocytes, qui sont infectées par le parasite sont transformées, ce qui se traduit par une leucémie chez l'animal. Les parasites purifiés de cellules infectées par *Theileria* expriment une protéine kinase CK2α. Or, la sous-unité CK2α interagirait avec PP2A pour moduler positivement son activité (Hériché H., et al, Regulation of Protein Phosphatase 2A by direct interaction with casein kinase 2α. Science 1997 ; 276 : 952-5). Aussi, la modulation de la PP2A via l'expression de la sous-unité CK2α pourrait être à la base du blocage de deux voies de signalisation dans la cellule parasitée, celle des MAP-kinases (Chaussepied M., et al. Theileria transformation of bovine leukocytes : a parasite model for the study of lymphoproliferation. Res Immunol. 1996 ; 147 : 127-38) et celle de la protéine kinase B (Akt) (M. Baumgartner, M. Chaussepied, MF Moreau, A. Garcia, G. Langsley. Constitutive PI3-K activity is essential for proliferation, but not survival, of Theileria parva - transformed B Cells. Cellular Micriobiol. (2000) 2, 329-339).

L'absence de motifs communs à l'ensemble des protéines interagissant avec PP2A empêche l'identification bio-informatique des motifs peptidiques directement impliqués dans la liaison de ces protéines avec PP2A.

Or, étant donné le rôle majeur des protéine phosphatases 2A dans les interactions virus-hôtes ou parasites-hôtes tel que résumé ci-dessus, on comprend l'intérêt d'identifier les sites de liaison des protéines virales ou parasitaires avec les holoenzymes PP2A ou l'une de leurs sous-unités, afin d'identifier de nouvelles cibles thérapeutiques pour ces pathogènes, viraux
ou parasitaires.

En particulier, l'identification des peptides interagissant avec PP2A permettrait de produire de nouveaux médicaments susceptibles de bloquer par inhibition compétitive les mécanismes cellulaires induits par les protéines virales ou parasitaires via leur interaction avec PP2A et en particulier les mécanismes d'infection, de prolifération des pathogènes et de transformation des cellules. Certains de ces peptides ont été, décrits dans W0-A- 9801563 (R. Marallus et al.).

L'invention s'intéresse à des moyens d'identifier des peptides de taille réduite, liant une holoenzyme PP2A ou l'une de ses sous-unités. Au contraire des protéines natives ou domaines polypeptidiques de taille importante, des peptides de taille réduite ont l'avantage d'être aisément synthétisés, par voie chimique ou en systèmes cellulaires, avec un rendement important et un coût réduit. Les peptides de l'invention sont en outre plus stables et plus facilement transférés dans le cytoplasme ou dans le noyau des cellules à l'aide de vecteurs appropriés, en vue d'une utilisation thérapeutique.

L'invention découle de la démonstration qu'il est possible d'identifier des peptides, d'une taille inférieure à 30 acides aminés, et notamment des peptides d'une taille inférieure à 20 acides aminés interagissant avec une holoenzyme PP2A ou l'une de ses sous-unités.

En particulier, les inventeurs ont montré que l'utilisation de la technique des « SPOT synthesis » décrites par Frank et Overwing (Methods in Molecular Biology, 1996, vol. 66 : 149-169, Epitope Mapping Protocols edited by : G.E. Morris Humana Press Inc., Totowa NJ) permet d'identifier les sites de liaison des protéines interagissant avec une holoenzyme PP2A ou l'une de ses sous-unités.

Les inventeurs ont par exemple identifié des peptides d'une taille inférieure à 20 acides aminés, interagissant *in vitro* avec l'holoenzyme PP2A purifiée ou l'une de ses sous-unités, lesdits peptides étant dérivés de la protéine *Vpr* de VIH-1 ou de la protéine CK2α du parasite *T. parva*. Des antagonistes dérivés de ces peptides et sélectionnés parce qu'ils inhibent l'interaction des protéines virales ou parasitaires avec une holoenzyme particulière de PP2A pourraient ainsi constituer de nouveaux agents antitumoraux, antiviraux ou antiparasitaires.

L'invention porte sur une méthode d'identification d'un peptide dont la séquence est issue d'une protéine virale, parasitaire ou cellulaire, ledit peptide liant spécifiquement une holoenzyme protéine phosphatase de type 2A ou l'une de ses sous-unités, ladite méthode comprenant les étapes consistant à :
a) déposer sous forme de spots, sur un support, des peptides dont la séquence est issue d'une protéine virale, parasitaire ou cellulaire, chaque spot correspondant au dépôt d'un peptide de séquence définie,
b) mettre en contact le support solide avec une solution contenant une holoenzyme protéine phosphatase 2A ou l'une de ses sous-unités dans des conditions permettant aux peptides présents sur le support, de lier l'holoenzyme ou l'une de ses sous-unités, et,
c) à identifier sur le support solide, le peptide sur lequel se fixe la protéine phosphatase 2A ou l'une de ses sous-unités.

Selon l'étape a), différents peptides sont déposés sur un support solide à des positions définies (« spot »), chaque position correspondant à une séquence peptidique spécifique et l'ensemble formant ainsi un réseau de peptides (« array ») à deux dimensions. Différentes méthodes de préparation de tels réseaux ont été décrites récemment (pour revue, Figeys et Pinto, 2001 Electrophoresis 22 : 208-216 ; Walter et al., 2000 Curr Opin Microbiol 3 : 298-302). L'ensemble de ces méthodes comprend en général la fixation covalente de peptides sur un support, en particulier à l'aide de lieurs (linkers) chimiques. A titre d'exemple, l'homme du métier pourra notamment se reporter à la technique des « SPOT synthesis » consistant à synthétiser directement sur une membrane de cellulose, des peptides comprenant jusqu'à 20 résidus (Frank et Overwing, Methods in Molecular Biology, 1996, vol. 66 : 149-169, Epitope Mapping Protocols edited by: G.E. Morris Humana Press Inc., Totowa NJ).

De manière générale, toute méthode peut être utilisée dès lors que celle-ci permet l'obtention d'un réseau de peptides déposés sur un support solide, utilisable pour détecter des interactions spécifiques entre les peptides déposés et des composés particuliers.

De façon très préférée, l'ensemble des séquences de peptides déposés recouvre la séquence complète de la protéine virale, parasitaire ou cellulaire dont ces séquences sont issues. Ainsi, le procédé permet de tester en une seule étape la séquence complète d'une protéine donnée, celle-ci étant « sectionnée » en un nombre fini de peptides, de séquences généralement chevauchantes.

Dans un mode de réalisation préféré, les peptides déposés sous forme de spot sont d'une taille inférieure à 20 acides aminés, et mieux, d'une taille inférieure à 15 acides aminés.

Dans un autre mode de réalisation particulier, les peptides sont déposés sur une membrane de cellulose.

Le réseau ainsi obtenu est mis en contact à l'étape b), avec une holoenzyme protéine phosphatase de type 2A ou l'une de ses sous-unités.

Par « holoenzyme protéine phosphatase de type 2A », il faut comprendre tout complexe dimérique (AC) ou hétérotrimérique (ABC), purifié d'un extrait cellulaire ou reconstitué après purification des deux sous-unités (A) et (C) d'une protéine phosphatase de type (2A) et le cas échéant d'une sous-unité (B). Les protéine phosphatases de type (2A) sont de préférence issues de mammifères.

Les supports sont incubés par exemple dans une solution tampon comprenant les protéine phosphatase purifiées ou l'une de leurs sous-unités purifiées. Une solution tampon utilisable est le TBS (TRIS BORATE) contenant 5% de régilait écrémé et 3% de BSA.

Le peptide sur lequel se fixe l'holoenzyme protéine phosphatase de type 2A est identifié en général par le marquage direct ou indirect de la protéine phosphatase et l'identification des spots au niveau desquels s'est fixé la protéine marquée. La fixation de la PP2A ou l'une de ses sous-unités au niveau de l'un des spots de peptides peut ainsi être révélée en particulier à l'aide d'antisérums, selon les techniques classiquement utilisées pour le Western Blot ou le test ELISA en phase solide, après incubation du support contenant le réseau de peptides avec un anticorps dirigé contre les sous-unités (A) ou (B) ou (C) ou un mélange d'anticorps dirigé contre les sous-unités (A), (B) ou (C) de PP2A.

L'application de la méthode de l'invention définie ci-dessus conduit à l'identification de peptides, notamment utiles dans le traitement de certaines infections virales ou parasitaires, d'une taille inférieure à 30 acides aminés, voire inférieure à 20 acides aminés, lesdits peptides étant capables de lier *in vitro* une holoenzyme protéine phosphatase de type 2A
ou l'une de ses sous-unités.

Dès lors, en utilisant ses connaissances générales dans le domaine de la synthèse peptidique, l'homme du métier peut produire des peptides, dérivés des fragments de peptides identifiés par la méthode de l'invention présentant les propriétés avantageuses décrites ci-dessus.

Par conséquent, la demande décrit un peptide, naturel ou synthétique, d'une taille inférieure à 30 acides aminés, de préférence inférieure à 20 acides aminés, **caractérisé en ce qu**'il lie *in vitro*, de manière spécifique, une holoenzyme protéine phosphatase de type 2A ou l'une de ses sous-unités, (A), (B) ou (C). Par liaison spécifique, il faut comprendre que le peptide est capable d'inhiber de manière compétitive la liaison d'une protéine d'origine virale ou parasitaire avec des PP2A.

Dans un mode de réalisation préféré ledit peptide est **caractérisé en ce qu**'il s'agit d'un fragment d'une protéine virale, parasitaire ou cellulaire, ladite protéine liant *in vitro* une protéine phosphatase de type 2A ou l'une de ses sous-unités, ou d'une séquence se distinguant du fragment de protéine précédent par substitution ou délétion d'acides aminés, ladite séquence distincte conservant néanmoins les propriétés de liaison à une protéine phosphatase de type 2A ou l'une de ses sous-unités. De préférence, le nombre d'acides aminés substitués ou supprimés dans la séquence distincte par rapport à la séquence initiale n'excède pas 20%, et mieux 10% du nombre d'acides aminés constituant la séquence initiale. De façon préférée, seuls les acides aminés dont la délétion n'affecte pas les propriétés de liaison *in vitro* du peptide à la PP2A sont substitués ou supprimés.

En particulier, une séquence distincte est une séquence peptidique augmentant l'affinité de liaison à la protéine phosphatase de type 2A ou l'une de ses sous-unités par rapport à la séquence dont elle dérive. Une autre séquence distincte telle que définie plus haut est une séquence peptidique homologue à une séquence peptidique identifiée initialement.Par séquence peptidique homologue, on entend dans la présente demande une séquence dérivée d'une protéine d'une autre espèce que la séquence peptidique identifiée initialement, et dont la séquence primaire peut être alignée avec la séquence peptidique identifiée initialement à l'aide d'un programme d'alignement optimal classiquement utilisé, tel que le programme BESTFIT (Wisconsin Genetics Software Package, Genetics Computer Group, GCG). En particulier, une séquence A sera considérée comme homologue à une séquence B si lesdites séquences A et B présentent au moins 50% d'identité, de préférence 75% d'identité, après alignement des séquences à l'aide d'un programme d'alignement optimal tel que le programme BESTFIT. De manière encore préférée, deux séquences sont aussi considérées homologues si les séquences sont quasi-identiques à l'exception de quelques résidus pouvant représenter 10 à 20% de variabilité sur la séquence totale. Par ailleurs les acides aminés de même fonction chimique (telles que par exemple, Arg et Lys) sont considérés comme équivalents. Les peptides à analyser pour leur liaison avec une PP2A ou l'une de ses sous-unités, sont en général choisis parmi des fragments de protéines virales, parasitaires ou cellulaires, lesquels protéines ont été montrées interagir *in vivo* ou *in vitro* avec une protéine phosphatase de type 2A.

De telles protéines virales, parasitaires ou cellulaires sont en particulier choisies parmi l'une des protéines suivantes : antigène t de SV40 ou de polyôme, antigène moyen t de polyôme, sous unité de PP2A de type B (B, B', B"), CK2α, CaMIV, p70S6-kinase, Pak1/Pak3, Tap42/alpha 4, PTPA, Set/I1/I2-PP2A, E4orf4, *tau, Vpr* ou CD28, CCXR2 (récepteur de chemokine).

Un peptide préféré est un fragment de la protéine CD28. L'invention a pour objet les peptides constitués par les séquences PRRPGPTRKHY (SEQ ID NO :33) et (PRRPGPTRK)₂ (SEQ ID NO :34) correspondant respectivement aux peptides nommés FD2 et FD3 dont la capacité de pénétration intracellulaire et leurs effets sur la viabilité des cellules sont décrits ci-après dans la partie expérimentale.

La demande décrit également des séquences peptidiques se distinguant d'un fragment de la protéine CD28 par substitution ou délétion d'acides aminés, lesdites séquences distinctes conservant néanmoins les propriétés de liaison à la protéine phosphatase de type 2A ou l'une de ses sous-unités.

Un peptide particulièrement préféré est un fragment de la protéine *Vpr* du virus VIH, en particulier un fragment de la protéine Vpr du virus VIH-1 ou VIH-2, ou une séquence se distinguant du fragment de protéine précédent par substitution ou délétion d'acides aminés, ladite séquence distincte conservant néanmoins les propriétés de liaison à la protéine phosphatase de type 2A ou l'une de ses sous-unités. L'invention ne comprend pas le peptide, fragment de la protéine Vpr ayant la séquence suivante: LFIHFRIGCQHSRIGITRRRRVRDGSSRP* divulguée dans la base de données EMBL, numéro d'accession P89821. En revanche, l'utilisation dudit peptide dans le cadre des applications décrites ci-après, fait partie de la présente invention.

L'invention a également pour objet un peptide dérivé d'une protéine qui interagit avec la protéine phosphatase de type 2A, dérivé de la protamine, le peptide de séquence RRRRRRRSRGRRRRTY (SEQ ID NO : 41, nommé FD8).

La demande décrit également, à titre d'exemple de peptides dérivés d'une protéine qui interagit avec la protéine phosphatase de type 2A, dérivé de la protamine, une séquence se distinguant de SEQ ID NO: 41 par substitution ou délétion d'acides aminés, ladite séquence distincte conservant néanmoins les propriétés de liaison à la protéine phosphatase de type 2A ou l'une de ses sous-unités.

De manière encore préférée, un peptide selon l'invention est **caractérisé en ce qu**'il est choisi parmi l'une des séquences suivantes :
a) VEALIRILQQLLFIHFRI (SEQ ID NO : 1), ou
b) RHSRIGIIQQRRTRNG (SEQ ID NO : 2).

La demande décrit également un peptide **caractérisé en ce qu**'il est inclus dans l'une des séquences suivantes :
a) VEALIRILQQLLFIHFRI (SEQ ID NO :1),
b) RHSRIGIIQQRRTRNG (SEQ ID NO : 2), ou,
c) une séquence se distinguant de SEQ ID NO : 1 ou SEQ ID NO :2 par substitution ou délétion d'acides aminés, ladite séquence distincte conservant néanmoins les propriétés de liaison pour la protéine phosphatase de type 2A ou l'une de ses sous-unités.

Un peptide particulièrement préféré selon l'invention est un fragment du peptide SEQ ID NO :2, ledit fragment consistant en, ou comprenant le peptide de séquence RHSRIG (SEQ ID NO: 36) nommé FD9 dont la capacité de pénétration intracellulaire et l'effet sur la viabilité des cellules ont été décrits ci-après dans la partie expérimentale.

La demande décrit également un composé à ossature polypeptidique contenant un peptide selon l'invention tel que défini ci-dessus, ledit composé étant d'un poids moléculaire compris entre 10 et 150 Kdaltons et ayant la capacité de lier la protéine phosphatase 2A.

L'invention concerne également un polypeptide **caractérisé en ce qu**'il est constitué de la répétition d'un peptide selon l'invention.

Des exemples de tels polypeptides sont notamment des polymères du peptide RHSRIG et en particulier, le dimère (RHSRIG)₂ (SEQ ID NO :37) ou encore le trimère (RHSRIG)₃ (SEQ ID NO :37), respectivement nommés FD10 et FD11 dont la capacité de pénétration intracellulaire et l'effet sur la viabilité des cellules ont été décrits ci-après dans la partie expérimentale.

Parmi les peptides de séquences se distinguant de SEQ ID NO :1 ou SEQ ID NO :2 par substitution ou délétion d'acides aminés, on citera plus particulièrement les peptides dont la séquence est incluse dans l'une des séquences de la protéine Vpr des différents variants du type VIH-1, VIH-2 et de SIV, et correspondant aux séquences homologues chez ces variants de SEQ ID NO :1 ou SEQ ID NO : 2.

On peut citer notamment les séquences de l'invention VEALIRILQQLL (SEQ ID NO : 6), ALIRILQQLLFI (SEQ ID NO : 7), IRILQQLLFIHF (SEQ ID NO : 8), ILQQLLFIHFR (SEQ ID NO: 9), RHSRIGIIQQRR (SEQ ID NO: 10), SRIGIIQQRRTR (SEQ ID NO : 11) et IGIIQQRRTRNG (SEQ ID NO : 12) correspondant aux dodécapeptides identifiés comme liant la sous-unité A de PP2A.

Une séquence selon l'invention se distinguant de SEQ ID NO :2 par délétion ou substitution d'acides aminés est en particulier la séquence RHSRIGVTRQRRARNG (SEQ ID NO: 40), également nommée FD13 dans la partie expérimentale présentée ci-après.

Un peptide préféré selon l'invention est un peptide choisi parmi l'une des séquences SEQ ID NO :1 ou SEQ ID NO :2 et **caractérisé en ce que** son administration induit l'apoptose des cellules tumorales.

Une méthode de sélection de peptides susceptibles d'induire l'apoptose des cellules tumorales peut être réalisée par exemple au moyen du test de viabilité MTT décrit dans la partie expérimentale.

La demande décrit également un peptide caractérisé en ce qu'il dérive d'un fragment de la protéine CK2α. En particulier, le peptide, naturel ou synthétique, est caractérisé en ce qu'il dérive d'un fragment de la protéine CK2α du parasite *Theileria parva.*

De manière encore préférée, un peptide selon l'invention est caractérisé en ce qu'il est choisi parmi l'une des séquences suivantes :
a) RKIGRGKFSEVFEG (SEQ ID NO :3),
b) TVTKDCVIKILKPVKKKKIKREIKILQNL (SEQ ID NO: 4), et
c) KILRLIDWGLAEFYHP (SEQ ID NO: 5).

La demande décrit également un peptide **caractérisé en ce qu**'il est inclus dans l'une des séquences suivantes :
a) RKIGRGKFSEVFEG (SEQ ID NO :3),
b) TVTKDCVIKILKPVKKKKIKREIKILQNL (SEQ ID NO: 4),
c) KILRLIDWGLAEFYHP (SEQ ID NO: 5),
d) Une séquence homologue de SEQ ID NO :3, SEQ ID NO :4 ou SEQ ID NO :5 dérivée de *P. falciparum* ou *leishmania*, ou,
e) une séquence se distinguant des séquences mentionnées ci-dessus par substitution ou délétion d'acides aminés, ladite séquence distincte conservant néanmoins les propriétés de liaison à la protéine phosphatase 2A ou l'une de ses sous-unités et notamment la séquence TVTKDKCVIKILKPVKKKKIKREIKILQNL (SEQ ID NO : 43).

Parmi les peptides se distinguant des séquences SEQ ID NO : 3, NO: 4 ou NO :5, on peut citer notamment les séquences du site 1 (RKIGRGKFSEVFEG) (SEQ ID NO: 3), et notamment le peptide de séquence RKIGRGKFSEVF et le peptide de séquence IGRGKFSEVFEG
ou les séquences du site 2 (TVTKDKCVIKILKPVKKKKIKREIKILQNL) (SEQ ID NO : 4) notamment les peptides suivants :
TVTKDKCVIKIL (SEQ ID NO : 13),
TKDKCVIKILKP (SEQ ID NO : 14),
DKCVIKILKPVK (SEQ ID NO: 15),
CVIKILKPVKKK (SEQ ID NO : 16),
IKILKPVKKKKI (SEQ ID NO : 17),
ILKPVKKKKIKR (SEQ ID NO : 18),
KPVKKKKIKREI (SEQ ID NO : 19),
VKKKKIKREIKI (SEQ ID NO : 20),
KKKIKREIKILQ (SEQ ID NO : 21),
KIKREIKILQNL (SEQ ID NO: 22), et enfin les séquences du site 3 KILRLIDWGLAEFTHP (SEQ ID NO : 5) soit le peptide de séquence KILRLIDWGLAE (SEQ ID NO : 23), le peptide de séquence LRLIDWGLAEFY (SEQ ID NO: 24), ou le peptide de séquence LIDWGLAEFYHP (SEQ ID NO : 25).

Un exemple de peptide selon l'invention comprenant une séquence homologue à *T. parva* du site 3 de la protéine CK2α chez *P. falciparum* est le peptide RQKRLI (SEQ ID NO : 42). L'invention porte également sur des polymères du peptide RQKRLI et notamment le trimère (RQKRLI)₃ (SEQ ID NO :35) nommé FD7 dans la partie expérimentale.

La demande décrit également un peptide dérivé de la protéine CK2α du parasite *Theileria parva* **caractérisé en ce que** son administration réduit le développement parasitaire. La demande décrit également des peptides dérivés de la protéine tau. La séquence tau présente un motif correspondant au site de liaison de la protéine E4orf4 d'adénovirus. Dans le cas de la maladie d'Alzheimer, la protéine tau est régulée par la protéine phosphatase 2A. De tels peptides seraient donc utiles dans le traitement de la maladie d'Alzheimer.

Les peptides identifiés par la méthode de l'invention sont particulièrement utiles dans le traitement de certaines tumeurs, de certaines infections virales ou parasitaires. L'homme du métier peut sélectionner, à l'aide de tests de compétition de liaison, de nouveaux peptides, dérivés des séquences identifiées selon la méthode de l'invention, lesdits peptides inhibant de manière compétitive la liaison de la protéine native dont il dérive avec une holoenzyme PP2A ou l'une de ses sous-unités.

Ainsi, la demande décut également un peptide naturel ou synthétique, tel que défini plus haut, **caractérisé en ce qu**'il inhibe de manière compétitive, l'interaction de la protéine native dont il dérive avec une holoenzyme PP2A ou l'une de ses sous-unités.

Les peptides selon l'invention, pour être efficace *in vivo* dans le traitement de certaines tumeurs ou certaines infections virales ou parasitaires, peuvent être couplés à un vecteur capable de transférer ledit peptide dans une cellule eucaryote. Cependant, il est possible comme discuté ci-dessous que les peptides selon l'invention possèdent eux-mêmes la capacité de pénétrer dans les cellules et par conséquent ne nécessitent pas l'adjonction d'un vecteur.

L'invention porte naturellement sur les moyens permettant la synthèse des peptides de l'invention. En particulier, l'invention porte sur un polynucléotide **caractérisé en ce que** sa séquence consiste en la séquence codante d'un peptide selon l'invention. Des polynucléotides préférés sont les polynucléotides dont la séquence est choisie parmi l'une des séquences suivantes 
SEQ IDs NO : 26
(5'GTGGAAGCCTTAATAAGAATTCTGCAACAACTGCTGTTTATTCATTT
CAGAATT),
 
NO : 27
(5'CGACATAGCAGAATAGGCATTATTCAACAGAGGAGAACAAGAAATG
GA),
 NO: 28 (5'-AGGAAGATCGGAAGAGGGAAGTTCAGTGAAGTTTTTGAGGGA), NO : 29 
(5'ACAGTAACGAAGGATAAATGCGTAATAAAAATCCTAAAGCCTGTAAA
GAAGAAGAAAATCAAGAGAGAGATTAAGATTCTACAGAACCTA),
 ou NO : 30 (5'AAAATACTAAGGCTAATTGACTGGGGATTAGCTGAGTTTTACCACCC A), codant respectivement les peptides NO : 1-5.

La demande décrit également des polynucléotides de séquences complémentaires à l'une des séquences SEQ ID NO :26-30 et les séquences hybridant dans des conditions stringentes auxdits polynucléotides.

Par « conditions stringentes », on entend les conditions qui permettent l'hybridation spécifique de deux séquences d'ADN simple brin à environ 65°C par exemple dans une solution de 6 x SSC, 0,5% SDS, 5X Denhardt's solution et 100 µg d'ADN carrier non spécifique ou toute autre solution de force ionique équivalente et après un lavage à 65°C, par exemple dans une solution d'au plus 0,2 x SSC et 0,1% SDS ou toute autre solution de force ionique équivalente. Les paramètres définissant les conditions de stringence dépendent de la température à laquelle 50% des brins appariés se séparent (Tm). Pour les séquences comprenant plus de 30 bases, Tm est définie par la relation : Tm = 81,5 + 0,41 (%G + C) +16,6Log (concentration en cations) - 0,63(% formamide) - (600/nombre de bases). Pour les séquences de longueur inférieure à 30 bases, Tm est définie par la relation : Tm = 4 (G+C) + 2(A+T). Les conditions de stringence sont également définies selon les protocoles décrits dans Sambrook *et al*., 2001 (Molecular Cloning : A laboratory Manual, 3rd Ed., Cold Spring Harbor, laboratory press, Cold Spring Harbor, New York).

II peut être avantageux de synthétiser un polypeptide comprenant la répétition des motifs peptidiques identifiés par le procédé de l'invention. Par conséquent, l'invention porte sur un polynucléotide **caractérisé en ce qu**'il consiste en un multimère du polynucléotide codant pour un peptide de l'invention. L'invention porte également sur un polypeptide **caractérisé en ce qu**'il est constitué de la répétition d'un peptide selon l'invention.

L'invention porte également sur un vecteur d'expression cellulaire, **caractérisé en ce qu**'il comporte un polynucléotide tel que défini plus haut et des séquences régulatrices permettant l'expression d'un peptide selon l'invention dans une cellule hôte.

L'invention vise également la méthode de préparation d'un peptide tel que défini selon l'invention, comprenant la transformation d'un hôte cellulaire à l'aide d'un vecteur d'expression cellulaire tel que défini plus haut, suivi de la mise en culture de l'hôte cellulaire ainsi transformé, et la récupération du peptide dans le milieu de culture.

L'invention porte en outre sur un anticorps polyclonal purifié ou un anticorps monoclonal **caractérisé en ce que** ledit anticorps est capable de lier de façon spécifique un peptide selon l'invention.

La demande décrit également un antisérum ou immunosérum **caractérisé en ce que** ledit antisérum ou immunosérum est capable de lier de façon spécifique un peptide selon l'invention.

Des anticorps spécifiquement dirigés contre les peptides identifiés par le procédé de l'invention sont obtenus, par exemple par immunisation d'un animal après injection d'un peptide selon l'invention, et récupération des anticorps produits. Un anticorps monoclonal peut être obtenu selon les techniques connues de l'homme du métier telle que la méthode des hybridomes décrites par Kohler et Milstein (1975).

Les anticorps obtenus, spécifiquement dirigés contre des cibles de la protéine phosphatase 2A trouvent leur application en particulier dans l'immunothérapie. Ils peuvent par exemple servir d'antagonistes de protéines virales ou parasitaires dirigés contre la protéine phosphatase 2A afin de bloquer le développement viral ou parasitaire.

De même, les polynucléotides codant les peptides de l'invention peuvent être directement transférés au noyau de cellules cibles, le cas échéant à l'aide de vecteurs appropriés, afin de permettre l'expression *in vivo* des peptides correspondants, lesdits peptides étant susceptibles de bloquer par inhibition compétitive une interaction spécifique entre la protéine phosphatase 2A et la protéine virale ou parasitaire dont ils dérivent.

Ainsi, l'invention porte sur une composition pharmaceutique comprenant un des éléments choisis parmi un polynucléotide selon l'invention ou un anticorps selon l'invention.

L'invention concerne également une composition pharmaceutique comprenant l'un des peptides de l'invention en combinaison avec un véhicule pharmaceutiquement acceptable.

L'invention vise en outre une utilisation d'un peptide de l'invention défini plus haut, dans la préparation d'un médicament utile dans le traitement d'une infection virale ou parasitaire.

L'invention vise préférentiellement l'utilisation d'un peptide de l'invention dont la séquence dérive d'un fragment de la protéine *Vpr*, tel que défini plus haut, dans la préparation d'un médicament apte à inhiber l'infection au VIH.

Les peptides de l'invention peuvent être avantageusement choisis de manière à stimuler l'induction de l'apoptose liée à l'activation de la protéine phosphatase 2A cellulaire. Ainsi, l'invention concerne également l'utilisation d'un peptide selon l'invention, tel que défini plus haut, dans la préparation d'un médicament apte à induire l'apoptose de cellules cibles, et en particulier de cellules tumorales.

Un autre aspect préféré de l'invention concerne l'utilisation d'un peptide de l'invention dont la séquence dérive d'un fragment de la protéine CK2α, dans la préparation d'un médicament apte à inhiber l'infection parasitaire. Plus particulièrement, l'invention vise l'utilisation d'un peptide de l'invention dans la préparation d'un médicament utile dans le traitement du paludisme.

L'infection virale ou parasitaire se traduit par une expression spécifique des protéines comprenant les séquences de peptides de l'invention. Les séquences codant les peptides de l'invention peuvent donc être utilisées comme sonde pour détecter, de manière spécifique, à partir d'ARN extrait d'un échantillon biologique d'un patient, une infection virale
ou parasitaire spécifique.

De même, un anticorps selon l'invention peut être utilisé pour reconnaître spécifiquement les séquences peptidiques contenues dans les protéines virales ou parasitaires exprimées lors de l'infection.

Ainsi, l'invention porte donc sur l'utilisation d'un polynucléotide selon l'invention ou d'un anticorps selon l'invention dans le diagnostic *in vitro* de pathologies parasitaires ou virales.

La demande décrict également la sélection et l'utilisation d'un peptide liant la protéine phosphatase 2A, et capable de pénétrer à l'intérieur des cellules.

Un exemple d'un tel peptide est illustré par le peptide FD6 (SEQ ID NO :20) dérivé de la protéine CK2α de *T. parva*. II a en effet été montré dans la présente invention que la présence de ce peptide dans la cellule n'affecte pas la viabilité de cellules de mammifères cultivées ou maintenues en survie.

La partie expérimentale qui suit illustre une application de la méthode d'identification des peptides de l'invention à l'identification de peptides issus de la protéine *Vpr* du VIH-1 et de la protéine CK2α du parasite *Theileria parva.* La demande décrit également la sélection et 25 l'utilisation d'un peptide liant la protéine phosphatase 2A et éventuellement capable de pénétrer à l'intérieur de la cellule, ledit peptide permettant de cibler et de mettre en contact avec la protéine phosphatase 2A intracellulaire une molécule capable de réguler l'activité de la protéine phosphatase 2A.

### DESCRIPTION DES FIGURES

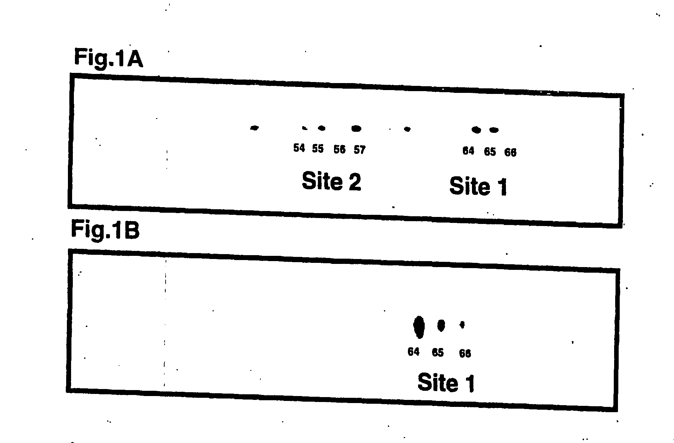

### Figure 1 : Criblage d'une membrane contenant des peptides recouvrant la séquence Vpr de HIV-1 avec la sous-unité structurale A de PP2A (A) et l'holoenzyme PP2A1 (B).

### Le recouvrement de la séquence des quatre peptides 54-57 définit la séquence du site 2 VEALIRILQQLLFIHFRI (SEQ ID NO : 1)

Peptide 54 : VEALIRILQQLL
Peptide 55 : ALIRILQQLLFI
Peptide 56 : IRILQQLLFIHF
Peptide 57 : ILQQLLFIHFRI

### Le recouvrement de la séquence des trois peptides 64 à 66 définit la séquence du site 1 RHSRIGIIQQRRTRNG (SEQ ID NO : 2)

Peptide 64 : RHSRIGIIQQRR
Peptide 65: SRIGIIQQRRTR
Peptide 66 : IGIIQQRRTRNG

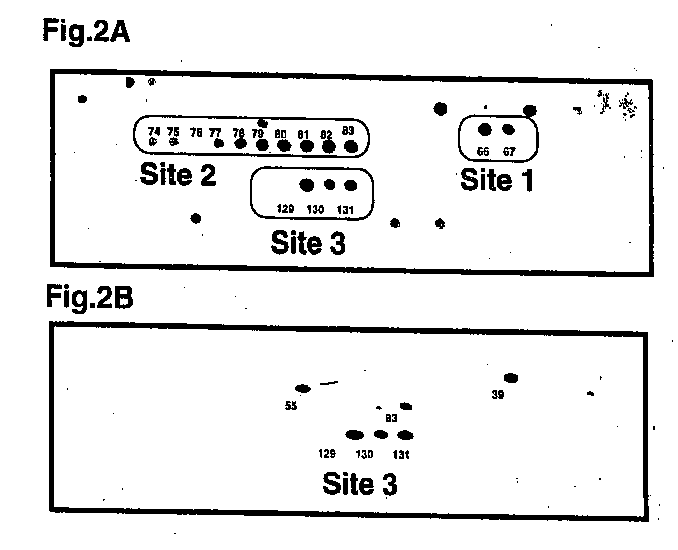

### Figure 2 : Criblage d'une membrane contenant des peptides recouvrant la séquence de CK2α de Theileria avec (A) la sous-unité structurale A de PP2A et (B) l'holoenzyme PP2A1.

### Le recouvrement de la séquence des deux peptides définit la sequence du site 1 RKIGRGKFSEVFEG (SEQ ID NO : 3)

Peptide 66 : RKIGRGKFSEVF
Peptide 67 : IGRGKFSEVFEG

### Le recouvrement de la séquence des dix peptides 74-83 définit la sequence du site 2 TVTKDKCVIKILKPVKKKKIKREIKILQNL

(SEQ ID NO : 4)
Peptide 74 : TVTKDKCVIKIL
Peptide 75 : TKDKCVIKILKP
Peptide 76 : DKCVIKILKPVK
Peptide 77 : CVIKILKPVKKK
Peptide 78 : IKILKPVKKKKI
Peptide 79 : ILKPVKKKKIKR
Peptide 80 : KPVKKKKIKREI
Peptide 81 : VKKKKIKREIKI
Peptide 82 : KKKIKREIKILQ
Peptide 83 : KIKREIKILQNL

### Le recouvrement de la séquence des trois peptides définit la sequence du site 3 KILRLIDWGLAEFTHP (SEQ ID NO : 5)

Peptide 129 : KILRLIDWGLAE
Peptide 130 : LRLIDWGLAEFY
Peptide 131 : LIDWGLAEFYHP

### Figure 3 : La figure 3 est un histogramme représentant les valeurs de pénétration intracellulaires obtenues à l'aide du test de pénétration cellulaire pour les peptides cités dans le tableau 3

### Figure 4 : La figure 4 illustre les effets de différents peptides sur la viabilité des cellules Héla évaluée à l'aide du test de viabilité MTT.

La viabilité des cellules Héla (exprimée en pourcentage par rapport à la population initiale) a été testée en présence de concentrations croissantes, respectivement de peptides FD8 (4A), FD13/FD14 (4B) et FD11/FD12 (4C).

### PARTIE EXPERIMENTALE

### A. Matériels et méthodes

### A.1 Les protéines PP2A purifiées

La protéine trimérique PP2A1 a été purifiée à homogénéité à partir de cerveau de porc.
Une sous-unité structurale recombinante de PP2A a été exprimée chez *E. coli* et purifiée selon le protocole décrit par Cohen et al. (Cohen P., Alemany S, Hemmings BA, Resink TJ, Stralfors P., Tung HY. Protein phosphatase-1 and protein phosphatase-2A from rabbit skeletal muscle. Methods Enzymol. 1988 159, 390-408), ou celui décrit par Bosch et al., (Bosch M, Cayla X, Van Hoof C, Hemmings BA, Ozon R., Merlevede W, Goris J. The PR55 and PR65 subunits of protein phosphatase 2A from Xenopus laevis. Molecular cloning and developmental regulation of expression. Eur J. Biochem. 1995 230,1037-45).

### A.2 -Méthode d'identification des sites de liaison de Vpr de VIH et CK2α de Theileria parva (T.parva )avec PP2A

Des peptides de liaison dérivés des protéines CK2α (codée par le protozoaire *T. parva)* ou Vpr (codée par le virus VIH-1) avec PP2A ont été identifiés en utilisant la technique des « peptides spot » précédemment décrite (Frank et Overwing. (1996). Meth. Mol. Biol. 66,149-169).

La méthode a consisté à synthétiser *in situ* sur une membrane de cellulose, à des positions définies, des dodécapeptides, dont l'ensemble des séquence recouvre la totalité de la séquence de la protéine d'intérêt (Vpr ou CK2α). Les peptides de deux spots consécutifs sur la membrane, se chevauchent avec un décalage de deux acides aminés.

Soixante huit (68) dodécapeptides recouvrant toute la séquence de la protéine Vpr de VIH-1 et deux cent cinq (205) dodécapeptides recouvrant la séquence de la protéine CK2α de *Theileria* ont été synthétisés et liés de façon covalente à des membranes de cellulose.

Chaque membrane ainsi préparée est d'abord saturée 1 heure à température ambiante avec du TBS contenant 5% de régilait écrémé et 3% de BSA puis incubée une nuit dans le même tampon en présence de 4µg/ml de protéine purifiée (sous-unité A de PP2A ou holoenzyme PP2A1).
L'interaction spécifique de chaque protéine purifiée (respectivement la sous-unité structurale A ou l'holoenzyme trimérique PP2A1) avec une séquence peptidique est révélée, comme un western blot, après incubation de la membrane avec un anticorps dirigé contre la protéine structurale A (Figures 1A et 2A) et avec un mélange d'anticorps reconnaissant les protéines A,B, et C de PP2A (Figures 1 B et 2B).

Les membranes sont lavées 5 fois 15 minutes avec un tampon classique TBST (TBS + TWEEN), utilisé pour l'incubation puis à nouveau incubées 1 heure à température ambiante avec un second anticorps (couplé à la péroxydase). Enfin, les membranes sont lavées 5 fois 15 minutes avec le tampon TBST et révélées.

### A3 Test de pénétration cellulaire

### 1- Cellules

Nous avons analysé la lignée Hela qui est dérivée d'un carcinome Cervical humain.

### 2- Dosages quantitatifs des peptides internalisés

### Tampon de lyse :

0,1 M tampon Tris pH 8 contenant 0,5 % NP40.

### Tampon OPD :

25,7 ml dibasic sodium phosphate 0,2 M + 24,3 ml acide citrique 0,1 M + 50 ml d'eau distillée ; ajuster pH 5,0.

### Complexes peptides biotinylés-avidine. :

4 moles de peptides sont incubées avec 1 mole d'avidine-péroxydase. 20 minutes à température ambiante

### -Analyse de la pénétration intracellulaire des divers peptides dans la cellule Hela

Les cellules Hela (10⁴ pour 100 I) sont ensemencées dans des plaques de 96 puits (fonds plats) avec du milieu complet DMEM en présence de 2,5 % pénicilline/ampicilline et de 10 % sérum de veau foetal. Après une nuit d'incubation à 37°c dans une étuve à C02 (5%), on ajoute différentes dilutions de complexes (peptides biotinylés-avidine peroxydase). Après 4 heures d'incubation le surnageant est aspiré, et les cellules lavées 3 fois avec du PBS, trypsinées et reprises pour numération dans 1 ml de PBS.
Après numération, les cellules sont reprises dans 300 µl de tampon de lyse.
- Mesure de l'activité peroxydase
   Dans une plaque ELISA 96 trous on incube 50 µl de tampon OPD avec 50 µl de tampon lyse ou 50 µl de lysat cellulaire (en général on effectue différentes dilutions successives (au 1/2)). Pour révéler, on ajoute (à l'obscurité ) 50 µl de la solution d'OPD. La réaction (environ 10min) est arrêtée avec 100 µl d'HCL 1 N.
- Analyse du résultat
   L'activité peroxydase est déterminée par lecture à 490 nm au lecteur ELISA (filtre de référence à 620 nm) et la quantité de peroxydase dans les lysats est calculée d'après la courbe de référence puis rapportée au même nombre de cellules (10³ ou 10⁴) :
   Molécules de peptides = (6 * 10²³ / PM du peptide) * ng de PO*10⁻⁹.

### A4 Test de viabilité cellulaire

Les cellules Hela (10⁴ pour 100µl) sont ensemencées dans des plaques de 96 puits (fonds plats) avec du milieu complet DMEM contenant 2,5 % pénicilline/ampicilline et 10 % sérum de veau foetal. Après une nuit d'incubation à 37°c dans une étuve à C02, et les cellules sont cultivées en présence de différentes concentrations de peptides. Après 72h d'incubation, le milieu contenant les peptides est aspiré et le MTT à 0,5 mg/ml (dilué dans du DMEM seul) est ajouté à raison de 100 µl par puits. L'incubation est réalisée dans le noir à 37°C pendant 30 minutes puis le MTT est aspiré et 50 µl de DMSO est ajouté dans tous les puits. Il faut attendre une dizaine de minutes pour la lyse complète des cellules et bien remuer le lysat pour homogénéiser la dissolution du produit de réaction dans le puits. Les plaques sont ensuite lues à 570 nm avec un filtre de référence à 690 nm.

### B. RESULTATS ET DISCUSSION

### B.1 Identification de séquences peptidiques contenant des sites de liaison de protéines codées par deux agents pathogènes (VIH-1 et T.parva) avec des PP2A (PP2A1 et sous-unité A).

Les résultats obtenus après l'incubation des membranes contenant les peptides recouvrant les séquences de Vpr de VIH-1 et CK2α de *T.parva* avec l'holoenzyme PP2A trimérique purifiée ont permis de déterminer cinq séquences de peptides de Vpr et de CK2α capables de lier spécifiquement PP2A et présentées dans le tableau ci-après :

**Tableau 1 Séquences peptidiques contenant des sites de liaison de HIV-1-Vpr et CK2α avec les PP2A**

| | | **Sous-unité A** | **PP2A1** |
|---|---|---|---|
| **HIV-1 *Vpr*** | **Site 1** | RHSRIGIIQQRRTRNG | RHSRIGIIQQRRTRNG |
| | **Site 2** | VEALIRILQQLLFIHFRI | |
| ***T.parva* Ck2** α | **Site 1** | RKIGRGKFSEVFEG | |
| | **Site 2** | TVTKDKCVIKILKPVKKKKIKREIKILQNL | |
| | **Site 3** | KILRLIDWGLAEFYHP | KILRLIDWGLAEFYHP |

Plus précisément, deux séquences peptidiques contenant un site de liaison de Vpr de VIH-1 avec la protéine PP2A1 (Fig.1B. "site 1 ") et avec la sous-unité A (Fig.1A "site 1" et "site 2") ont été identifiées. Trois séquences peptidiques contenant un site de liaison de CK2α de *T. parva* avec la protéine PP2A1 (Fig.2B "site 3") et avec la sous-unité structurale A ont aussi été identifiées (Fig.2A" site 1", " site 2" et "site 3").

### B.2 Intérêt de l'utilisation des peptides de Vpr de VIH-1 qui lient PP2A

L'expression, exogène ou due à l'infection provirale, de Vpr de VIH-1 induit l'apoptose des cellules HeLa, des lignées lymphoïdes T et des lymphocytes primaires (Stewart et al., 1997 J Virol 71: 5579-9). L'utilisation de mutants Vpr a dans un premier temps permis de corréler cet effet à l'arrêt des cellules en phase G2 du cycle cellulaire. Plus récemment on a montré que Vpr peut aussi induire l'apoptose indépendemment de l'arrêt en G2 (Nishizawa et al. 2000 Virology 27: 16-26).

Il a été rapporté que l'activation de PP2A après interaction avec la protéine adénovirale E4orf4, induit l'apoptose dans les cellules transformées (Shtrichman R et al., 2000 Oncogene 19: 3757-3765). De façon analogue l'expression de Vpr induit aussi l'apoptose dans les cellules transformées (Stewart et al. 1999, PNAS 96: 12039-12043).

Par ailleurs, l'analyse des mutants de Vpr connus dans l'état de la technique indique que les peptides identifiés par le procédé de l'invention et liant spécifiquement la protéine PP2A, contiennent des séquences qui corrèlent avec celles requises pour l'effet pro-apoptotique de Vpr.

Ainsi, les fragments des protéines virales, Vpr et E4orf4 qui interagissent avec PP2A et identifiés par le procédé de l'invention pourraient être utiles pour induire l'apoptose des cellules tumorales.

Les peptides identifiés sont aussi naturellement utiles dans l'inhibition de l'infection par le VIH, voire d'autres virus et rétrovirus apparentés.

### B.3 Intérêt de l'utilisation des séquences de CK2 α de T.parva qui lient PP2A

L'utilisation de l'acide okadaïque et de l'antigène petit t de SV40 a permis de démontrer que la PP2A contrôle la prolifération cellulaire via une nouvelle cascade de phosphorylations impliquant la PI3-kinase, la PKC ξ (identifiée comme une MAP-Kinase-Kinase-Kinase ou MEKK), la protéine MEK et les deux MAP-Kinases ERK-1 et ERK-2 et les facteurs de transcription NF-κB et Sp1 (Sontag,E., Sontag,J.M., Garcia.A. (1997). EMBO.J.,16, 5662-5671; Ayllón, V., Martinez-A., C., Garcia, A., Cayla, X. and Rebollo, A (2000). EMBO J. 19, 1-10, A.Garcia, S.Cereghini., E.Sontag (2000). J.Biol Chem. 275 :9385-9389). Par ailleurs le rôle de PP2A dans la régulation de la cascade des MAP-kinases a aussi été suggéré par les travaux de l'équipe de Chambaz (Hériché et al. (1997), Science, 276: 952-955) qui ont montré que la surexpression de la sous-unité CK2α cellulaire active PP2A qui déphosphoryle la protéine Mek.

Les travaux de Ole-Moi et coli. (EMBO J. (1993) 12: 1621-1631) ont montré que la transformation par *Theileria* induit une hyperphosphorylation des protéines de l'hôte. Cet effet est en partie dû à l'activation constitutive de la CK2 cellulaire qui serait elle même dépendante de l'action d'une sous unité de type CK2α codée par le parasite et sécrétée dans le cytosol de la cellule transformée.

Comme indiqué ci-dessous, la comparaison des séquences identifiées par le procédé de l'invention correspondant aux trois sites de liaison avec PP2A permet d'identifier la présence d'un motif du type: **K-I-G/L-R/K** qui est partiellement répété dans le site 2

Site 1 : KIGR

Site 2 : KILKPVKKKKIKREKILQNL,

Site 3 : KIL RLI (duplication partielle KIL/RLI).

Il est intéressant de noter que le site de liaison de l'ATP de CK2α recouvre partiellement le site 1 et le site 2, ce qui suggère une inhibition de l'activité kinase après interaction de CK2α avec la sous-unité A. Par ailleurs, comme on peut le constater dans le tableau 2 ci-après, les trois séquences contenant les sites de liaison de CK2α de *T. parva* avec PP2A sont conservées parmi plusieurs espèces comprenant les parasites *P.Falciparum* et *Leishmania*.

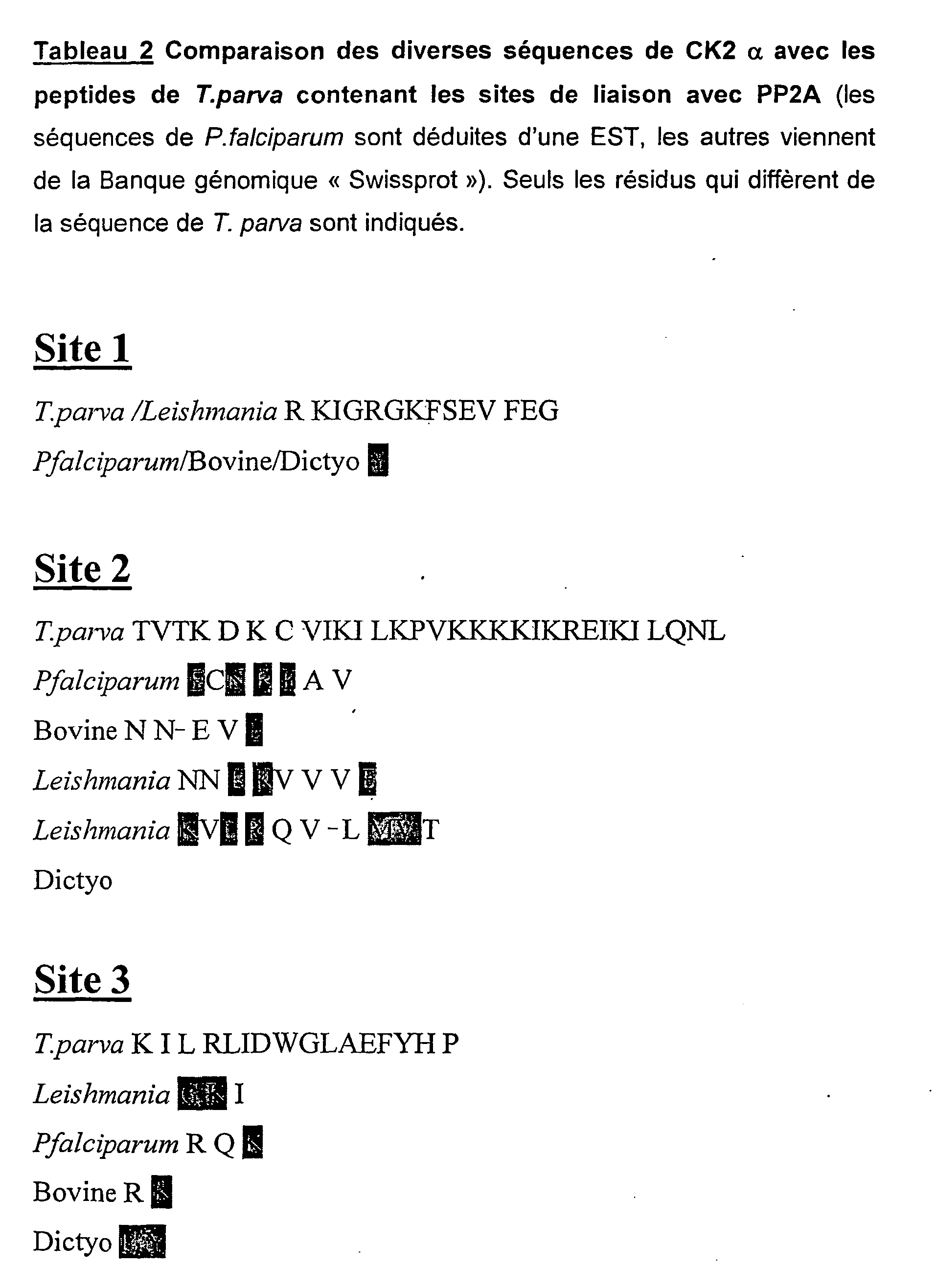

L'analyse fine des interactions suggère que les CK2α de ces différentes espèces devraient interagir avec PP2A; par exemple le peptide 131 de CK2α de *T.parva* décrit à la figure 2 et dans lequel les quatre premiers acides aminés du site 3 sont délétés est capable de lier PP2A.
Ceci suggère que les CK2α de *Leishmania*, de *P.falciparum* qui diffèrent pour les 3 premiers acides aminés devraient lier PP2A. Ceci est consistant avec le fait que le motif KILRLI présente une duplication de K/R-II/L-I/L qui, dans un contexte basique, pourrait être un site de liaison pour PP2A.

La présence, à l'intérieur de la cellule de ces peptides, correspondant aux sites de liaison *in vivo* des protéines avec PP2A, pourrait donc contrarier le développement de ces parasites.

### B.4 Effets biologiques des composés peptidiques selon l'invention sur les cellules

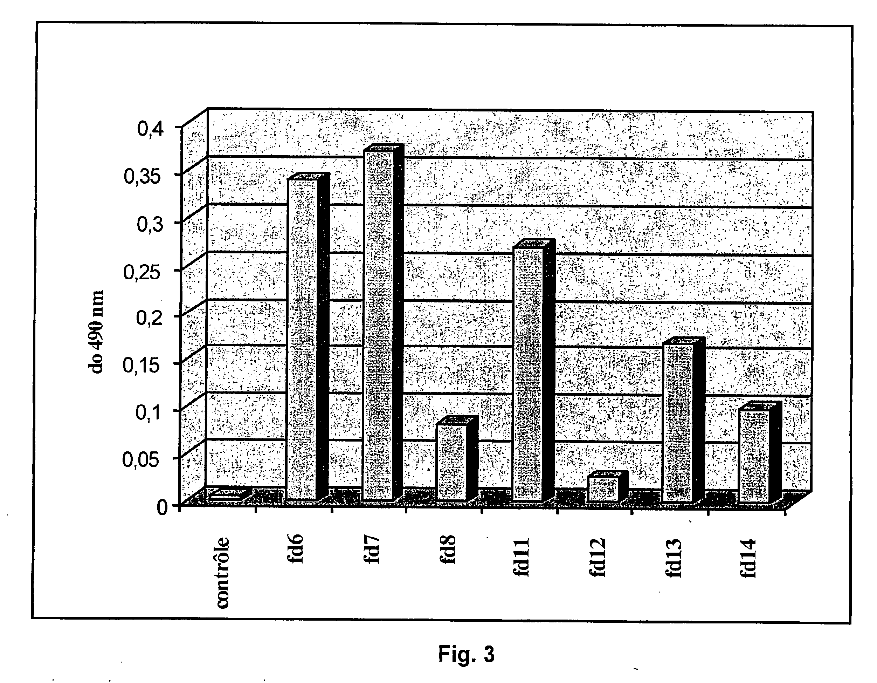

Les divers peptides listés dans le tableau 3 ont été synthétisés sous forme biotinylés, purifiés par HPLC (Neosystem) et leur effet sur la pénétration intracellulaire et viabilité cellulaire a été analysé dans les cellules Hela.
L'étude de l'ensemble des peptides repertoriés dans le tableau 1 a permis de determiner six peptides qui ont la possibilité de pénétrer dans la cellule cellule Hela (Fig.3).
- Fd6 : un peptide de 12AA dérivé d'un site d'interaction de la sous-unité A de PP2A avec la protéine CK2α de *T.parva.*
- Fd7 : un peptide de 18AA correspondant à trois répétitions d'un hexa motif de 6AA ; cette séquence, dérivée de la CK2α de *P.Falciparum, est* homologue à la séquence de *T. parva* qui lie PP2A.
- Fd8 : un peptide dérivé de la protamine (un activateur connu des PP2A).
- Fd11 : un peptide de 18AA correspondant à trois répétitions d'un hexa motif de 6AA dérivé de la séquence de FD14.
- Fd14 : FD14 qui reproduit le site de liaison que nous avons caractérisé de HIV-1 Vpr avec PP2A.
- Fd13: Ce peptide correspond à une séquence de HIV-1 Vpr qui est homologue à la séquence du peptide FD14 qui représente un site de liaison avec PP2A avec un autre HIV-1 Vpr.

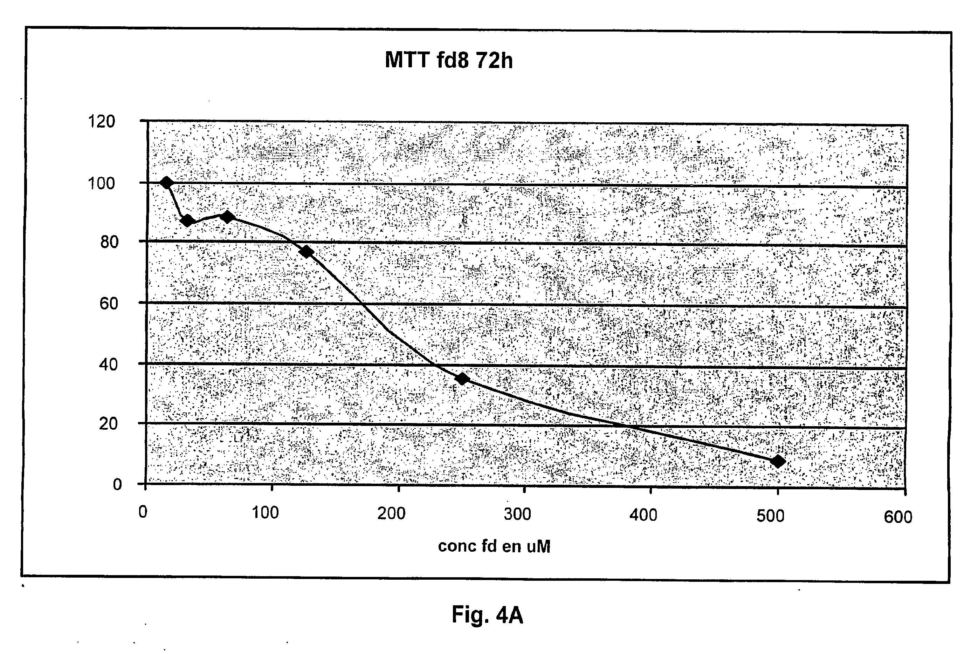

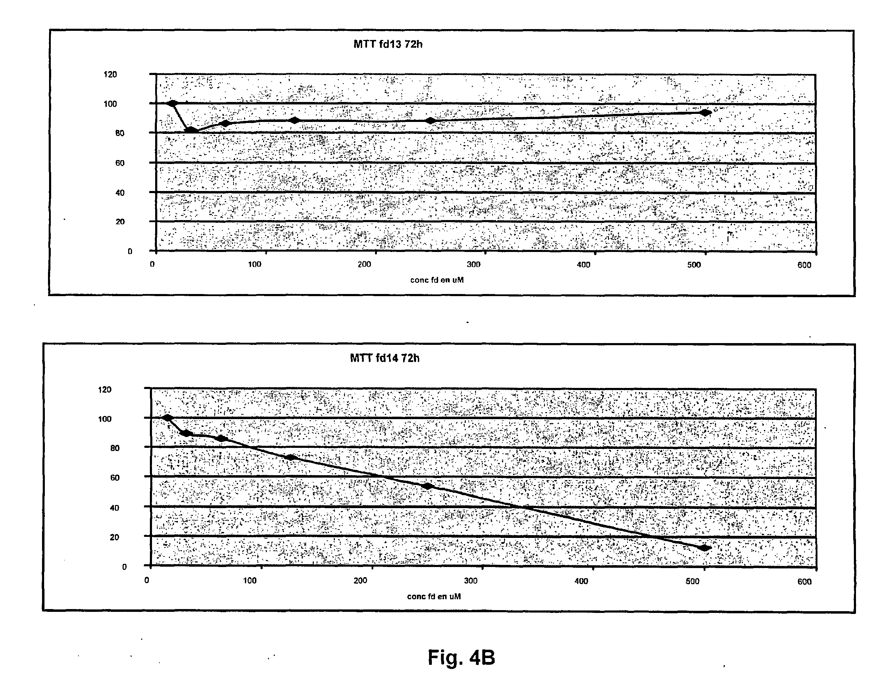

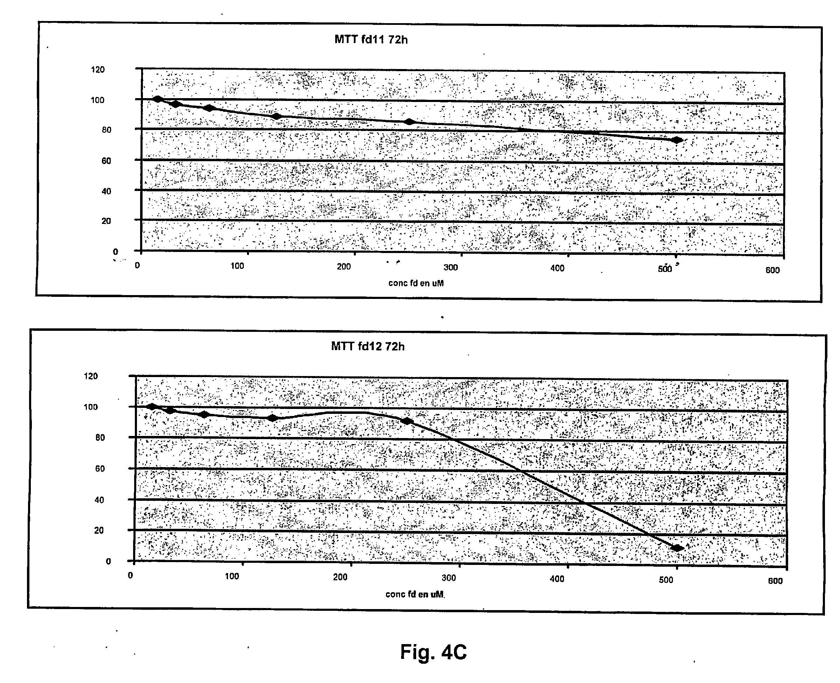

Par ailleurs, les études de viabilité effectuées sur l'ensemble des peptides répertoriés dans le tableau 1 a permis d'identifier trois peptides qui inhibent la viabilité des cellules Hela.
- Fd8 : affecte la viabilité des cellules Hela (Figure 4A)
- Fd14 : affecte clairement la viabilité des cellules Hela (Figure 4B)
- Fd12 : un peptide de 18AA dont la séquence dérive de celle du peptide FD11 (Le R est muté en A). Ce peptide qui est homologue à la proteine glucosamine transférase de *Chlamydia muridarum* affecte la viabilité de la cellule Hela (figure 4C). Cet effet biologique pourrait être dû à une interaction avec la membrane plasmique.

**Tableau 3 : Peptides mimant des sites de liaison de protéines cibles avec des PP2A**

| **Protéines d'origine** | **Code peptides** | **Séquences peptides** | **Seq ID NO:** |
|---|---|---|---|
| **CD28** | | | |
| | FD2 | -PRRPGPTRKHY | Seq ID NO:132 |
| | FD3 | -(PRRPGPTRK)2 | Seq ID NO:133 |
| **CK2α *T. parva*** | | | |
| | FD6 | -VKKKKIKREIKI | Seq ID NO: 20 |
| **CK2α *P.Falciparum* (analogue *T.parva*)** | FD7 | -(RQKRLI)3 | Seq ID NO:134 |
| **Vpr (HIV-1)** | | | |
| | FD9 | -RHSRIG | Seq ID NO:135 |
| | FD10 | -(RHSRIG)2 | Seq ID NO:136 |
| | FD11 | -(RHSRIG)3 | Seq ID NO:137 |
| | FD12* | -(AHSRIG)3 (FD11 mutation R- -A) | Seq ID NO:138 |
| | FD13 | RHSRIGVTRQRRARNG (analogue FD14) | Seq ID NO:139 |
| | FD14 | RHSRIGIIQQRRTRNG | Seq ID NO:2 |
| **Protamine** | FD8 | RRRRRRRSRGRRRRTY | Seq ID NO:140 |

### Discussion

Les peptides issus de certaines protéines qui interagissent avec des PP2A : une nouvelle approche anti-tumorale ?

Notre étude a permis d'identifier deux peptides pénétrants (FD8 /FD14) dérivés de deux protéines ,Vpr et protamine, connues pour interagir avec des PP2A. Ces peptides qui ont en commun des séquences riche en Argine ou en lysine, pourraient donc pénétrer dans la cellule en utilisant un mécanisme général d'internalisation. Un tel mécanisme, commun pour l'internalisation des peptides ayant des séquences riche en Argine, a été récemment proposé (Tomoki Suzuki, et al(2002), Possible Existence of Common Internalization Mechanisms among Arginine-rich Peptides, JBC 277:2437-2443). De façon générale la présence de séquences riches en Argine ou en lysine caracterise les proteines liant la PP2A ce qui suggère que d'autres peptides pénétrants pourraient être identifiés dans la famille des PP2A.

Vpr, une protéine codée par le virus VIH-1, est impliquée dans le maintien d'une charge virale élevée et dans l'établissement de la pathogenèse liée au VIH. L'expression de Vpr, exogène ou due à l'infection provirale de HIV-1, induit l'apoptose dans des cellules HeLa, dans des lignées lymphoïdes T, dans des lymphocytes primaires et dans les cellules transformées (Stewart et al.J Virol 1997 ; 71 : 5579-9 ; Stewart et al. 1999, PNAS, 96, 12039-12043). Par ailleurs II a été rapporté que l'interaction de PP2A avec une autre protéine virale, Adenovirus E4orf4 (Marcellus et al .J Virol. (2000) 74:7869-7877) peut induire l'apoptose des cellules tumorales. Au total ces résultats suggèrent l'hypothèse que l'activation de certaines PP2A serait un nouveau moyen d'induire l'apoptose des tumeurs. Dans ce sens, nos résultats présentés à la figure 4B suggèrent que le peptide F14 dérivé de HIV-1 Vpr pourrait représenter un biopepide anti-tumoral. L'absence d'effet biologique du peptide FD13 (dont la séquence diffère de quatre AA par rapport à FD14-tableau 2) suggère que la structure de FD14 est critique pour réguler la viabilité de Hela. Par conséquent, l'obtention de molécules chimiques mimant la structure du peptide FD14 pourrait donc permettre de générer de nouvelles substances anti-tumorales.

## Revendications

1. Peptide **caractérisé en ce qu'**il s'agit de la séquence peptidique PRRPGPTRKHY (SEQ ID NO : 33).

2. Peptide **caractérisé en ce qu'**il est choisi parmi l'une des séquences peptidiques suivantes :
RRRRRRRSRGRRRRTY (SEQ ID NO: 41);
VEALIRILQQLLFIHFRI (SEQ ID NO:1);
RHSRIGIIQQRRTRNG (SEQ ID NO:2);
VEALIRILQQLL (SEQ ID NO : 6);
ALIRILQQLLFI (SEQ ID NO : 7);
IRILQQLLFIHF (SEQ ID NO : 8);
ILQQLLFIHFRI (SEQ ID NO : 9);
RHSRIGVTRQRRARNG (SEQ ID NO : 40);
RHSRIGIIQQRR (SEQ ID NO : 10);
IIQQRRTR (SEQ ID NO : 11);
QQRRTRNG (SEQ ID NO : 12); et
RHSRIG (SEQ ID NO : 36).

3. Peptide **caractérisé en ce qu'**il est choisi parmi l'une des séquence suivantes :
RKIGRGKFSEVFEG (SEQ ID NO: 3);
TVTKDCVIKILKPVKKKKIKREIKILQNL (SEQ ID NO: 4);
KILRLIDWGLAEFYHP (SEQ ID NO: 5);
RKIGRGKFSEVF (SEQ ID NO: .31);
IGRGKFSEVFEG (SEQ ID NO : 32);
TVTKDKCVIKIL (SEQ ID NO : 13);
TKDKCVIKILKP (SEQ ID NO : 14);
DKCVIKILKPVK (SEQ ID NO : 15);
CVIKILKPVKKK (SEQ ID NO : 16);
IKILKPVKKKKI (SEQ ID NO : 17);
ILKPVKKKKIKR (SEQ ID NO : 18);
KPVKKKKIKREI (SEQ ID NO : 19);
VKKKKIKREIKI (SEQ ID NO : 20);
KKKIKREIKILQ (SEQ ID NO : 21);
KIKREIKILQNL (SEQ ID NO : 22);
TVTKDKCVIKILKPVKKKKIKREIKILQNL (SEQ ID NO :43);
KILRLIDWGLAE (SEQ ID NO : 23) ;
LRLIDWGLAEFY (SEQ ID NO : 24);
LIDWGLAEFYHP (SEQ ID NO : 25); et
RQKRLI (SEQ ID NO: 42).

4. Peptide selon l'une des revendications 1 à 3, **caractérisé en ce qu'**il est couplé à un vecteur capable de transférer ledit peptide dans une cellule eucaryote.

5. Polypeptide **caractérisé en ce qu'**il est constitué de la répétition d'un peptide selon l'une des revendications 1 à 3.

6. Polypeptide selon la revendication 5, **caractérisé en ce qu'**il est choisi parmi l'une des séquences suivantes :
(RHSRIG)₂ (SEQ ID NO : 37);
(RHSRIG)₃ (SEQ ID NO : 38);
(AHSRIG)₃ (SEQ ID NO: 39);
(PRRPGPTRK)₂ (SEQ ID NO : 34); et
(RQKRLI)₃ (SEQ ID NO : 35).

7. Polynucléotide **caractérisé en ce que** sa séquence consiste en la séquence codante d'un peptide selon l'une quelconque des revendications 1 à 6.

8. Polynucléotide **caractérisé en ce que** sa séquence est choisie parmi l'une des séquences suivantes SEQ ID NO : 26, NO : 27, NO : 28, NO : 29 ou NO : 30.

9. Polynucléotide **caractérisé en ce qu'**il consiste en un multimère du polynucléotide selon la revendication 7 ou 8.

10. Vecteur d'expression cellulaire, **caractérisé en ce qu'**il comporte un polynucléotide selon l'une des revendications 7 à 9 et des séquences régulatrices permettant l'expression d'un peptide selon l'une quelconque des revendications 1 à 6 dans une cellule hôte.

11. Anticorps purifié polyclonal ou monoclonal **caractérisé en ce qu'**il est capable de lier de façon spécifique l'un quelconque des peptides selon l'une des revendications 1 à 6.

12. Composition pharmaceutique comprenant l'un des peptides selon l'une des revendications 1 à 6, en combinaison avec un véhicule pharmaceutiquement acceptable.

13. Composition pharmaceutique comprenant un des éléments choisis parmi un polynucléotide selon l'une des revendications 7 à 9, un vecteur d'expression selon la revendication 10 ou un anticorps selon la revendication 11.

14. Peptide, **caractérisé en ce qu'**il est choisi parmi l'une des séquences suivantes :
- (RHSRIG)₃ (SEQ ID NO: 38);
- RHSRIGVTRQRRARNG (SEQ ID NO: 40);
- RRRRRRRSRGRRRRTY (SEQ ID NO: 41).

15. Peptide **caractérisé en ce qu'**il s'agit de la séquence VKKKKIKREIKI (SEQ ID NO: 20).

16. Utilisation d'un peptide ou polypeptide défini selon l'une des revendications 1 à 6, 14 ou 15 ou du peptide de séquence LFIHFRIGCQHSRIGITRRRRVRDGSSRP (SEQ ID NO: 44), dans la préparation d'un médicament utile dans le traitement d'une infection virale ou parasitaire.

17. Utilisation d'un peptide ou polypeptide défini selon l'une des revendications 2, 14 ou 15 ou du peptide de séquence LFIHFRIGCQHSRIGITRRRRVRDGSSRP (SEQ ID NO: 44), dans la préparation d'un médicament apte à inhiber l'infection au VIH.

18. Utilisation d'un peptide défini selon l'une des revendications 2 à 6 ou 14 ou du peptide de séquence LFIHFRIGCQHSRIGITRRRRVRDGSSRP (SEQ ID NO: 44), dans la préparation d'un médicament apte à induire l'apoptose de cellules cibles, et en particulier de cellules tumorales.

19. Utilisation d'un peptide défini selon la revendication 3, dans la préparation d'un médicament apte à inhiber l'infection parasitaire.

20. Utilisation d'un peptide défini selon la revendication 3, dans la préparation d'un médicament utile dans le traitement du paludisme.

21. Utilisation d'un polynucléotide selon l'une des revendications 7 à 9 ou d'un anticorps selon la revendication 11, dans le diagnostic *in vitro* de pathologies parasitaires ou virales.

22. Méthode d'identification d'un peptide dont la séquence est issue d'une protéine virale, parasitaire ou cellulaire, ledit peptide liant spécifiquement une holoenzyme protéine phosphatase de type 2A ou l'une de ses sous-unités, ladite méthode comprenant les étapes consistant à :
a) déposer sous forme de spots, sur un support, des peptides dont la séquence est issue d'une protéine virale, parasitaire ou cellulaire, chaque spot correspondant au dépôt d'un peptide de séquence définie,
b) mettre en contact le support solide avec une solution contenant l'holoenzyme protéine phosphatase 2A ou l'une de ses sous-unités, dans des conditions permettant aux peptides présents sur le support, de lier l'holoenzyme ou l'une de ses sous-unités, et,
c) à identifier sur le support solide, le peptide sur lequel se fixe la protéine phosphatase 2A ou l'une de ses sous-unités.

23. Méthode selon la revendication 22, **caractérisée en ce que** les peptides déposés sous forme de spot sont d'une taille inférieure à 20 acides aminés, de préférence inférieure à 15 acides aminés.

24. Méthode selon la revendication 22 ou 23, **caractérisée en ce que** les peptides sont déposés sur une membrane de cellulose.

25. Méthode selon l'une quelconque des revendications 22 à 24, **caractérisée en ce que** l'ensemble des peptides déposés recouvre la séquence complète de la protéine virale, parasitaire ou cellulaire dont les séquences sont issues.

26. Méthode de préparation d'un peptide tel que défini selon l'une quelconque des revendications 1 à 6, 14 ou 15, comprenant la transformation d'un hôte cellulaire à l'aide d'un vecteur d'expression cellulaire tel que défini à la revendication 10, suivi de la mise en culture de l'hôte cellulaire ainsi transformé, et la récupération du peptide dans le milieu de culture.

## Claims

1. Peptide **characterized in that** it is the peptide sequence PRRPGPTRKHY (SEQ ID No: 33).

2. Peptide **characterized in that** it is selected from one of the following peptide sequences:
RRRRRRRSRGRRRRTY (SEQ ID No. 41);
VEALIRILQQLLFIHFRI (SEQ ID No. 1);
RHSRIGIIQQRRTRNG (SEQ ID No.2);
VEALIRILQQLL (SEQ ID No. 6);
ALIRILQQLLFI (SEQ ID No. 7);
IRILQQLLFIHF (SEQ ID No. 8);
ILQQLLFIHFRI (SEQ ID No. 9);
RHSRIGVTRGRRARNG (SEQ ID No. 40);
RHSRIGIIQQRR (SEQ ID No. 10);
IIQQRRTR (SEQ ID No. 11);
QQRRTRNG (SEQ ID No. 12); and
RHSRIG (SEQ ID No. 36).

3. Peptide **characterized in that** it is selected from one of the following sequences:
RKIGRGKFSEVFEG (SEQ ID No. 3);
TVTKDCVIKILKPVKKKKIKREIKILQNL (SEQ ID No. 4);
KILRLIDWGLAEFYHP (SEQ ID No. 5);
RKIGRGKFSEVF (SEQ ID No. 31);
IGRGKFSEVFEG (SEQ ID No. 32);
TVTKDKCVIKIL (SEQ ID No. 13);
TKDKCVIKILKP (SEQ ID No. 14);
DKCVIKILKPVK (SEQ ID No. 15);
CVIKILKPVKKK (SEQ ID No. 16);
IKILKPVKKKKI (SEQ ID No. 17);
ILKPVKKKKIKR (SEQ ID No. 18);
KPVKKKKIKREI (SEQ ID No. 19);
VKKKKIKREIKI (SEQ ID No. 20);
KKKIKREIKILQ (SEO ID No. 21);
KIKREIKILQNL (SEQ ID No. 22);
TVTKDKCVIKILKPVKKKKIKREIKILQNL (SEQ ID No. 43);
KILRLIDWGLAE (SEQ ID No. 23);
LRLIDWGLAEFY (SEQ ID No. 24);
LIDWGLAEFYHP (SEQ ID No. 25); and
RQKRLI (SEQ ID No. 42).

4. Peptide according to one of Claims 1 to 3, **characterized in that** it is coupled to a vector capable of transferring said peptide into a eukaryotic cell.

5. Polypeptide **characterized in that** it is constituted of the repetition of a peptide according to one of Claims 1 to 3.

6. Polypeptide according to Claim 5, **characterized in that** it is selected from one of the following sequences:
(RHSRIG)₂ (SEQ ID No. 37);
(RHSRIG)₃ (SEQ ID No. 38);
(AHSRIG)₃ (SEQ ID No.39);
(PRRPGPTRK)₂ (SEQ ID No. 34); and
(RQKRLI)₃ (SEQ ID No. 35).

7. Polynucleotide **characterized in that** the sequence thereof consists of the coding sequence of a peptide according to any one of Claims 1 to 6.

8. Polynucleotide **characterized in that** the sequence thereof is selected from one of the following sequences: SEQ ID No. 26, No. 27, No. 28, No. 29 or No. 30.

9. Polynucleotide **characterized in that** it is made up of a multimer of the polynucleotide according to Claim 7 or 8.

10. Cell expression vector, **characterized in that** it comprises a polynucleotide according to one of Claims 7 to 9 and regulatory sequences for the expression of a peptide according to any one of Claims 1 to 6 in a host cell.

11. Purified polyclonal or monoclonal antibody, **characterized in that** it is capable of specifically binding any one of the peptides according to one of Claims 1 to 6.

12. Pharmaceutical composition comprising one of the peptides according to one of Claims 1 to 6, in combination with a pharmaceutically acceptable vehicle.

13. Pharmaceutical composition comprising one of the elements selected from a polynucleotide according to one of Claims 7 to 9, an expression vector according to Claim 10 or an antibody according to Claim 11.

14. Peptide **characterized in that** it is selected from one of the following sequences:
- (RHSRIG)₃ (SEQ ID No. 38);
- RHSRIGVTRQRRARNG (SEQ ID No. 40);
- RRRRRRRSRGRRRRTY (SEQ ID No. 41).

15. Peptide **characterized in that** it is the sequence VKKKKIKREIKI (SEQ ID No. 20).

16. Use of a peptide or polypeptide defined according to one of Claims 1 to 6, 14 or 15 or of the peptide of sequence LFIHFRIGCQHSRIGITRRRRVRDGSSRP (SEQ ID No. 44), in the preparation of a medicament for use in the treatment of a viral or parasitic infection.

17. Use of a peptide or polypeptide defined according to one of Claims 2, 14 or 15 or of the peptide of sequence LFIHFRIGCQHSRIGITRRRRVRDGSSRP (SEQ ID No. 44), in the preparation of a medicament capable of inhibiting HIV infection.

18. Use of a peptide defined according to one of Claims 2 to 6 or 14 or of the peptide of sequence LFIHFRIGCQHSRIGITRRRRVRDGSSRP (SEQ ID No. 44), in the preparation of a medicament capable of inducing target-cell apoptosis, and in particular tumour-cell apoptosis.

19. Use of a peptide defined according to Claim 3, in the preparation of a medicament capable of inhibiting parasitic infection.

20. Use of a peptide defined according to Claim 3, in the preparation of a medicament for use in the treatment of malaria.

21. Use of a polynucleotide according to one of Claims 7 to 9 or of an antibody according to Claim 11, in the in vitro diagnosis of parasitic or viral pathologies.

22. Method for identifying a peptide of which the sequence is derived from a viral, parasitic or cell protein, said peptide specifically binding a type 2A protein phosphatase holoenzyme or a subunit thereof, said method comprising the steps consisting in:
a) depositing, in the form of spots, on a support, peptides of which the sequence is derived from a viral, parasitic or cell protein, each spot corresponding to the deposit of a peptide of defined sequence,
b) bringing the solid support into contact with a solution containing the protein phosphatase 2A holoenzyme or a subunit thereof, under conditions which allow the peptides present on the support to bind the holoenzyme or a subunit thereof, and
c) identifying, on the solid support, the peptide to which the protein phosphatase 2A or a subunit thereof binds.

23. Method according to Claim 22, **characterized in that** the peptides deposited in the form of a spot are less than 20 amino acids in size, preferably less than 15 amino acids in size.

24. Method according to Claim 22 or 23, **characterized in that** the peptides are deposited on a cellulose membrane.

25. Method according to any one of Claims 22 to 24, **characterized in that** the collection of peptides deposited covers the complete sequence of the viral, parasitic or cell protein from which the sequences are derived.

26. Method for preparing a peptide as defined according to any one of Claims 1 to 6, 14 or 15, which comprises transforming a cellular host using a cell expression vector as defined in Claim 10, followed by culturing the cellular host thus transformed, and recovering the peptide from the culture medium.

## Patentansprüche

1. Peptid, **dadurch gekennzeichnet, daß** es die Peptidsequenz PRRPGPTRKHY (SEQ ID NR.: 33) umfasst.

2. Peptid, **dadurch gekennzeichnet, daß** es ausgewählt ist aus der Gruppe bestehend aus den folgenden Peptidsequenzen:
RRRRRRRSRGRRRRTY (SEQ ID NR.: 41);
VEALIRILQQLLFIHFRI (SEQ ID NR.: 1);
RHSRIGIIQQRRTRNG (SEQ ID NR.: 2);
VEALIRILQQLL (SEQ ID NR.: 6);
AIRILQQLIFI (SEQ ID NR.: 7);
IRILQQLLFIHF (SEQ ID NR.: 8);
ILQQLLFIHFRI (SEQ ID NR.: 9);
RHSRIGVTRQRRARNG (SEQ ID NR.: 40);
RHSRIGIIQQRR (SEQ ID NR.: 10);
IIQQRRTR (SEQ ID NR.: 11);
QQRRTRNG (SEQ ID NR. :12); und
RHSRIG (SEQ ID NR.: 36).

3. Peptid, **dadurch gekennzeichnet, daß** es ausgewählt ist unter den folgenden Sequenzen:
RKIGRGKFSEVFEG (SEQ ID NR.: 3);
TVTKDCVIKILKPVKKKKIKPEIKILQNL (SEQ ID NR.: 4);
KILRLIDWGLAEFYHP (SEQ ID NR.: 5);
RIGGRGKFSEVF (SEQ ID NR.: 31);
IGRGKFSEVFEG (SEQ ID NR.: 32);
TVTKDKCVIKIL (SEQ ID NR.: 13);
TKDKCVIKILKP (SEQ ID NR.: 14);
DKCVIKILKPVK (SEQ ID NR.: 15);
CVIKILKPVKKK (SEQ ID NR.: 16);
IKILKPVKKKKI (SEQ ID NR.: 17);
ILKPVKKKKIKR (SEQ ID NR.: 18);
KPVKKKKIKREI (SEQ ID NR.: 19);
VKKKKIKREIKI (SEQ ID NR.: 20);
KKKIKREIKILQ (SEQ ID NR.: 21);
KIKREIKILQNL (SEQ ID NR.: 22);
TVTKDKCVIKILKPVKKKKIKPEIKILQNL (SEQ ID NR.: 43);
KILRLIDWGLAE (SEQ ID NR.: 23);
LRLIDWGLAEFY (SEQ ID NR.: 24);
LIDWGLAEFYHP (SEQ ID NR.: 25); und
RQKRLI (SEQ ID NR.: 42).

4. Peptid nach einem der Ansprüche 1 bis 3, **dadurch gekennzeichnet, daß** es mit einem Vektor verbunden ist, der das Peptid in eine eukaryotische Zelle transferieren kann.

5. Polypeptid, **dadurch gekennzeichnet, daß** es eine Wiederholung eines Peptids nach einem der Ansprüche 1 bis 3 darstellt.

6. Polypeptid nach Anspruch 5, **dadurch gekennzeichnet, daß** es ausgewählt ist unter den folgenden Sequenzen:
(RHSRIG)₂ (SEQ ID NR.: 37);
(RHSIRIG)₃ (SEQ ID NR.: 38);
(AHSRIG)₃ (SEQ ID NR.: 39);
(PRRPGPTRK)₂ (SEQ ID NR.: 34); und
(RQKRLI)₃ (SEQ ID NR. : 35).

7. Polynukleotid, **dadurch gekennzeichnet, daß** seine Sequenz aus einer Sequenz besteht, die ein Peptid nach einem der Ansprüche 1 bis 6 kodiert.

8. Polynukleotid, **dadurch gekennzeichnet, daß** seine Sequenz ausgewählt ist unter den folgenden Sequenzen SEQ ID NR.: 26, NR.: 27, NR.: 28, NR.: 29 oder NR.: 30.

9. Polynukleotid, **dadurch gekennzeichnet, daß** es aus einem Multimer des Polynukleotids nach Anspruch 7 oder 8 besteht.

10. Zellulärer Expressionsvektor, **dadurch gekennzeichnet, daß** er ein Polynukleotid nach einem der Ansprüche 7 bis 9 umfasst, und daß die Regulationssequenz die Expression eines Peptids nach einem der Ansprüche 1 bis 6 in einem zellulären Wirt ermöglicht.

11. Gereinigter polyklonaler oder monoklonaler Antikörper, **dadurch gekennzeichnet, daß** er spezifisch an ein Peptid nach einem der Ansprüche 1 bis 6 bindet.

12. Pharmazeutische Zusammensetzung, die ein Peptid nach einem der Ansprüche 1 bis 6 in Kombination mit einem pharmazeurisch verträglichen Träger umfasst.

13. Pharmazeutische Zusammensetzung, die ein Element umfasst ausgewählt unter einem Polynukleotid nach einem der Ansprüche 7 bis 9, einem Vektor nach Anspruch 10, oder einem Antikörper nach Anspruch 11.

14. Peptid, **dadurch gekennzeichnet, daß** es ausgewählt ist unter einer der folgenden Sequenzen:
(RHSRIG)₃ (SEQ ID NR.: 38);
RIISRIGVTRQRRARNG (SEQ ID NR.: 40);
RRRRRRRSRGRRRRTY (SEQ ID NR.: 41).

15. Peptid, **dadurch gekennzeichnet, daß** es die Sequenz VKKKKIKREIKI (SEQ ID NR. 20) umfasst.

16. Verwendung eines Peptids oder Polypeptids definiert in einem der Ansprüche 1 bis 6, 14 oder 15, oder eines Peptids der Sequenz LFIHFRIGCQHSRIGITRRRRVRDGSSRP (SEQ ID NR. 44) zur Herstellung eines Medikaments zur Behandlung einer Virus- oder Parasiten-Infektion.

17. Verwendung eines Peptids oder Polypeptids definiert in einem der Ansprüche 2, 14 oder 15, oder eines Peptids der Sequenz LFIHFRIGCQHSRIGITRRRRVRDGSSRP (SEQ ID NR. 44) zur Herstellung eines Medikaments zur Inhibierung einer HIV-Infektion.

18. Verwendung eines Peptids nach einem der Ansprüche 2 bis 6 oder 14 oder eines Peptids der Sequenz LFIHFRIGCQHSRIGITRRRRVRDGSSRP (SEQ ID NR. 44) zur Herstellung eines Medikaments zur Induzierung der Apoptose von bestimmten Zellen, insbesondere von Tumorzellen.

19. Verwendung eines Peptids nach Anspruch 3 zur Herstellung eines Medikaments zur Inhibierung einer Parasiteninfektion.

20. Verwendung eines Peptids nach Anspruch 3 zur Herstellung eines Medikaments zur Behandlung von Malaria.

21. Verwendung eines Polynukleotids nach einem der Ansprüche 7 bis 9 oder eines Antikörpers nach Anspruch 11 zur in vitro Diagnose von parasitären oder viralen Erkrankungen.

22. Verfahren zu Identifizierung eines Peptids, dessen Sequenz von einem viralen, parasitären oder zellulären Protein abgeleitet ist, und das spezifisch an ein Holoenzym Proteinphosphatase des Typs 2A oder an eines seiner Untereinheiten bindet, wobei das Verfahren die folgenden Schritte umfasst:
a) Auftragen der Peptide, deren Sequenz abgeleitet ist von einem viralen, parasitären oder zellulären Protein in Form von Spots auf einen Träger, wobei jeder Spot einer Menge eines Peptids mit definierter Sequenz entspricht,
b) Inkontaktbringen des festen Trägers mit einer Lösung, die das Holoenzym Proteinphosphatase 2A oder eine seiner Untereinheiten umfasst, wobei die Bedingungen dem auf dem Träger vorhandenen Peptid ermöglichen, an das Holoenzym oder eine seiner Untereinheiten zu binden, und
c) Identifizieren des Peptids auf dem festen Träger, an das die Proteinphosphatase 2A oder eine seiner Untereinheiten gebunden hat.

23. Verfahren nach Anspruch 22, **dadurch gekennzeichnet, daß** die in Form eines Spots aufgetragenen Peptide eine Größe von weniger als 20 Aminosäuren, vorzugsweise weniger als 15 Aminosäuren aufweisen.

24. Verfahren nach Anspruch 22 oder 23, **dadurch gekennzeichnet, daß** die Peptide auf einer Zellulosemembran aufgetragen wurden.

25. Erfahren nach einem der Ansprüche 22 bis 24, **dadurch gekennzeichnet, daß** alle aufgetragenen Peptide zusammen die vollständige Sequenz des viralen, parasitären oder zellulären Proteins, von denen die Sequenzen abgeleitet wurden, abdecken.

26. Verfahren zur Herstellung eines Peptids nach einem der Ansprüche 1 bis 6, 14 oder 15, welches umfasst, Transformieren eines zellulären Wirts mittels eines zellulären Expressionsvektors nach Anspruch 10, und anschließend Züchten des so transformierten zellulären Wirts, und gewinnen des Peptids im Kulturmedium.
